# EUROPEAN PATENT APPLICATION

(11) **EP 4 755 446 A1**
(43) Date of publication of application: **10.06.2026**
(21) Application number: 25217367.9
(22) Date of filing: 20.11.2025
(51) Int. Cl.: A61N 5/10

(54) **SYSTEMS AND METHODS FOR CONFIGURING A PROTON BEAM SYSTEM TO DELIVER ENERGY TO A PATIENT**

(30) Priority: 03.12.2024 US 202418966951
(71) Applicant: Siemens Healthineers International AG, 6312 Steinhausen (CH)
(72) Inventor: MAGLIARI, Anthony, Newark, 60541 (US); LANSONNEUR, Pierre, 69004 Lyon (FR); KRIEGER, Miriam, 5235 Rüfenach (CH); FOLKERTS, Michael, Costa Mesa, 92626 (US)
(74) Representative: Mathisen & Macara LLP

(57) **Abstract**

Provided herein are systems (200) for configuring a proton beam system to deliver energy to a patient. In some examples, systems (200) can include one or more processors that are configured to receive (302) scan data associated with a three-dimensional scan of a patient, determine (304) a treatment plan involving delivery of energy from a plurality of gantry positions relative to the patient, and provide (308) control data associated with the treatment plan to a proton beam system, the control data configured to control the proton beam system when executing the treatment plan. In some examples, each field specified by the treatment plan can be associated with a beam configuration that targets the planning target volume such that each spot path (420) of the beam configuration is separated from at least one other spot path (420) of the beam configuration by a distance that satisfies a threshold distance.

## Description

### TECHNICAL FIELD

This application relates generally to systems and methods for configuring a proton beam system to deliver energy to a patient and, in some embodiments, to systems and methods for configuring a proton beam system to deliver energy to a patient using beam configurations that reduce or eliminate doses delivered to organs at risk or other tissue not included in a planning target volume.

### BACKGROUND

Radiation therapy (also referred to as "radiotherapy" or "RT") involves the delivery of radiation (energy) to targets within the body of a patient during treatment. For example, a proton beam system can be configured to move relative to a patient in accordance with an RT treatment plan to multiple control points and deliver energy to target tissue (e.g., tumors) to treat cancer located within the body of the patient. These control points can be bound by fields, and the proton beam system can cause one or more beams to be transmitted toward the patient in a grid pattern with these fields. This can allow for the delivery of energy toward the target tissue, with the goal of eliminating the target tissue without affecting the surrounding tissue.

But conventional methods for developing treatment plans to treat the patient can be difficult to optimize. For example, due to the ways in which conventional proton therapy treatment plans are developed, and the ways in which each proton beam interacts with the patient when targeting a planning target volume (PTV), energy is often delivered to organs at risk (OARs), causing distress or damage to such tissue. Further, conventional treatment planning platforms can be limited when delivering FLASH radiotherapy using proton beams. For example, conventional approaches can be restricted to a limited number of beams, which in turn can compromise the dosimetric quality compared to the volumetric modulated arc therapy (VMAT) treatment modalities. Additionally, overlapping proton beam paths can lead to a potential reduction in the FLASH effect, and can impact therapeutic benefits provided by the treatment.

Conventional techniques for generating proton therapy treatment plans can also be constrained by the need for extensive beam overlap and sequential "back and forth" delivery of spot patterns in pencil beam scanning (PBS). As a result, the maximum PTV target size can be limited, and dose rate calculations used to deliver desired amounts of energy to a target can increase significantly in complexity to achieve desired doses delivered to the PTV while minimizing energy delivered to OARs. Further, as PBS generally involves continuous dose monitoring from the first point in time at which energy is delivered to a voxel until a final point in time, significant computing resources may be needed to plan for, and perform, a given proton beam treatment plan. These combined issues can reduce the efficiency and effectiveness of proton-based FLASH radiotherapy.

### SUMMARY

For the aforementioned reasons, there is a need for systems and methods that improve upon the generation of treatment plans to configure medical devices such as proton beam systems when treating patients. Moreover, there is a need for a more innovative approach to beam arrangement and dose delivery.

In accordance with a first aspect of the invention, there is provided a system for optimizing dose delivery when configuring a proton beam system to deliver energy to target a planning target volume, the system as defined by claim 1.

In accordance with a second aspect of the invention, there is provided a method for optimizing dose delivery when configuring a proton beam system to deliver energy to target a planning target volume, the method as defined by claim 7.

In accordance with a third aspect of the invention, there is provided a non-transitory computer-readable medium storing instructions, as defined by claim 13.

Optional features are defined by the dependent claims.

The methods and systems discussed herein can eliminate range uncertainty by configuring the transmission of proton beams along a plurality of spot paths (where the Bragg peak is placed outside the target or PTV) such that interference (e.g., re-irradiation) between the spot paths of a given field is reduced or eliminated. As a result, treatment plans can be developed where each spot path is transmitted at a significantly higher level (e.g., maximum power levels for some proton beam systems) as compared to conventional treatment plans without negatively impacting desirable FLASH effects. Further, treatment plans developed in accordance with the techniques described herein can minimize or eliminate irradiation of portions of a patient along a beam path beyond the targeted OAR (e.g., in cases where the Bragg peak is not placed outside the patient). These benefits, in turn, can allow for faster treatment times, faster recovery of the patient in areas where dose delivery is intended to be minimized, and reduced complication when generating and implementing proton therapy treatment plans. The treatment paradigm disclosed herein can also allow for treatment plans that have a sharp lateral penumbra in the plateau region, leading to more precise dose delivery to the PTV without significant impacts to OARs beyond the PTV. The described techniques can also reduce the biological impact of linear energy transfer (LET), simplifying treatment planning by eliminating the need for LET calculations and, likewise, eliminating the computational resources needed to perform these calculations.

### System

In an embodiment, a system for optimizing dose delivery when configuring a proton beam system to deliver energy to target a planning target volume (PTV) can include one or more processors configured to receive scan data associated with a three-dimensional scan of a patient, the three-dimensional scan representing at least a portion of a planning target volume. The one or more processors can be configured to determine a treatment plan involving delivery of energy from a plurality of gantry positions relative to the patient, where each gantry position corresponds to a field according to which at least a portion of the energy is configured to be delivered to the patient. In some implementations, the one or more processors can be configured to determine, for each field, a beam configuration to target the planning target volume such that each spot path of the beam configuration is separated from at least one other spot path of the beam configuration by a distance that satisfies a threshold distance. The one or more processors can be configured to update the treatment plan based on the beam configuration for each field. In some implementations, the one or more processors can be configured to provide control data associated with the treatment plan to a proton beam system, the control data configured to control the proton beam system when executing the treatment plan.

In some aspects, the one or more processors configured to determine the beam configuration for each field can be configured to assign each spot path of the beam configuration to a position within the field such that energy delivered to the patient by a first spot path of the beam configuration does not overlap with energy delivered to the patient by a second spot path of the beam configuration. In aspects, the one or more processors configured to determine the beam configuration for each field can be configured to assign each spot path of the beam configuration to a position within the field such that energy delivered to the patient by a first spot path of the beam configuration does not overlap with the energy delivered to the patient by a second spot path outside of a threshold distance associated with the PTV. In at least some aspects, the one or more processors configured to determine the beam configuration for each field can be configured to assign a plurality of spot paths to the beam configuration such that energy delivered to the patient by each spot path targets the PTV. The one or more processors can be configured to determine a set of spot paths that are configured to deliver energy to a location outside of a threshold distance associated with the PTV. In some implementations, the one or more processors can be configured to unassign a subset of spot paths included in the set of spot paths from the plurality of spot paths such that energy delivered by the plurality of spot paths does not overlap at the location outside of the threshold distance.

In some aspects, the one or more processors configured to determine the beam configuration for each field can be configured to assign a plurality of spot paths to the beam configuration such that energy delivered to the patient by each spot path targets the PTV, and determine a set of spot paths that are configured to deliver energy to a location outside of a threshold distance associated with the PTV. The one or more processors can be configured to unassign a subset of spot paths included in the set of spot paths from the plurality of spot paths such that energy delivered by the plurality of spot paths does not overlap at the location outside of the threshold distance. In aspects, the one or more processors configured to determine the beam configuration for each field can be configured to determine the beam configuration such that a set of spot paths of the beam configuration form pristine Bragg peaks along spot paths that run parallel to each other. In some aspects, the one or more processors configured to determine the beam configuration can be configured to determine the beam configuration such that a set of spot paths of the beam configuration form pristine Bragg peaks along spot paths that run parallel to each other and deliver a cumulative amount of energy that satisfies a threshold value at a location beyond the PTV along an axis defined by the set of spot paths. In aspects, the one or more processors configured to determine the beam configuration can be configured to determine the beam configuration such that a set of spot paths of the beam configuration form pristine Bragg peaks along spot paths that run parallel to each other and deliver energy associated with the pristine Bragg peaks outside of the patient.

### Method

In an embodiment, a method for optimizing dose delivery when configuring a proton beam system to deliver energy to target a planning target volume (PTV) is disclosed. The method can include receiving, by one or more processors, scan data associated with a three-dimensional scan of a patient, the three-dimensional scan representing at least a portion of a planning target volume; determining, by the one or more processors, a treatment plan involving delivery of energy from a plurality of gantry positions relative to the patient, where each gantry position corresponds to a field according to which at least a portion of the energy is configured to be delivered to the patient; and determining, for each field and by the one or more processors, a beam configuration configured to target the planning target volume such that each spot path of the beam configuration is separated from at least one other spot path of the beam configuration by a distance that satisfies a threshold distance. In some implementations, the method can include updating the treatment plan based on the beam configuration for each field. The method can include providing, by the one or more processors, control data associated with the treatment plan to a proton beam system, the control data configured to control the proton beam system when executing the treatment plan.

In some aspects, determining the beam configuration for each field can include assigning, by the one or more processors, each spot path of the beam configuration to a position within the field such that energy delivered to the patient by a first spot path of the beam configuration does not overlap with energy delivered to the patient by a second spot path of the beam configuration. In aspects, determining the beam configuration for each field can include assigning, by the one or more processors, each spot path of the beam configuration to a position within the field such that energy delivered to the patient by a first spot path of the beam configuration does not overlap with the energy delivered to the patient by a second spot path outside of a threshold distance associated with the PTV. In some aspects, the determining the beam configuration for each field can include assigning, by the one or more processors, a plurality of spot paths to the beam configuration such that energy delivered to the patient by each spot path targets the PTV; determining, by the one or more processors, a set of spot paths that are configured to deliver energy to a location outside of a threshold distance associated with the PTV; and unassigning, by the one or more processors, a subset of spot paths included in the set of spot paths from the plurality of spot paths such that energy delivered by the plurality of spot paths does not overlap at the location outside of the threshold distance.

In some aspects, determining the beam configuration for each field can include assigning, by the one or more processors, a plurality of spot paths to the beam configuration such that energy delivered to the patient by each spot path targets the PTV. The method can include determining, by the one or more processors, a set of spot paths that are configured to deliver energy to a location outside of a threshold distance associated with the PTV. In some implementations, the method can include unassigning, by the one or more processors, a subset of spot paths included in the set of spot paths from the plurality of spot paths such that energy delivered by the plurality of spot paths does not result in energy delivered beyond a threshold amount at the location outside of the threshold distance.

In some aspects, determining the beam configuration for each field can include determining, by the one or more processors, the beam configuration such that a set of spot paths of the beam configuration form pristine Bragg peaks along spot paths that run parallel to each other or run along divergent spot paths extending from the proton beam system. In aspects, the determining the beam configuration can include determining, by the one or more processors, the beam configuration such that a set of spot paths of the beam configuration form pristine Bragg peaks along spot paths that run parallel to each other and deliver a cumulative amount of energy that satisfies a threshold value at a location beyond the PTV along an axis defined by the set of spot paths. In some aspects, determining the beam configuration can include determining, by the one or more processors, the beam configuration such that a set of spot paths of the beam configuration form pristine Bragg peaks along spot paths that run parallel to each other and deliver energy associated with the pristine Bragg peaks outside of the patient.

### Medium

In yet another embodiment, a non-transitory computer-readable medium is disclosed. The non-transitory computer-readable medium can store instructions thereon that, when executed by one or more processors, cause the one or more processors to receive scan data associated with a three-dimensional scan of a patient, the three-dimensional scan representing at least a portion of a planning target volume (PTV); determine a treatment plan involving delivery of energy from a plurality of gantry positions relative to the patient, where each gantry position corresponds to a field according to which at least a portion of the energy is configured to be delivered to the patient, and determine, for each field, a beam configuration configured to target the planning target volume such that each spot path of the beam configuration is separated from at least one other spot path of the beam configuration by a distance that satisfies a threshold distance. The non-transitory computer-readable medium can include instructions that cause the one or more processors to update the treatment plan based on the beam configuration for each field. The instructions can cause the one or more processors to provide control data associated with the treatment plan to a proton beam system, the control data configured to control the proton beam system when executing the treatment plan.

In some aspects, the instructions that cause the one or more processors to determine the beam configuration for each field can cause the one or more processors to: assign each spot path of the beam configuration to a position within the field such that energy delivered to the patient by a first spot path of the beam configuration does not overlap with energy delivered to the patient by a second spot path of the beam configuration. In aspects, the instructions that cause the one or more processors to determine the beam configuration for each field can cause the one or more processors to: assign each spot path of the beam configuration to a position within the field such that energy delivered to the patient by a first spot path of the beam configuration does not overlap with the energy delivered to the patient by a second spot path outside of a threshold distance associated with the PTV. In some aspects, the instructions that cause the one or more processors to determine the beam configuration for each field can cause the one or more processors to: assign a plurality of spot paths to the beam configuration such that energy delivered to the patient by each spot path targets the PTV; determine a set of spot paths that are configured to deliver energy to a location outside of a threshold distance associated with the PTV; and unassign a subset of spot paths included in the set of spot paths from the plurality of spot paths such that energy delivered by the plurality of spot paths does not overlap at the location outside of the threshold distance, or such that energy delivered by the plurality of spot paths does not result in energy delivered beyond a threshold amount at the location outside of the threshold distance.
In some aspects, the instructions that cause the one or more processors to determine the beam configuration for each field can cause the one or more processors to: determine the beam configuration such that a set of spot paths of the beam configuration form pristine Bragg peaks along spot paths that run parallel to each other.
In some aspects, the instructions that cause the one or more processors to determine the beam configuration for each field can cause the one or more processors to: determine the beam configuration such that a set of spot paths of the beam configuration form pristine Bragg peaks along spot paths that run parallel to each other and deliver a cumulative amount of energy that satisfies a threshold value at a location beyond the PTV along an axis defined by the set of spot paths.
In some aspects, the instructions that cause the one or more processors to determine the beam configuration for each field can cause the one or more processors to: determine the beam configuration such that a set of spot paths of the beam configuration form pristine Bragg peaks along spot paths that run parallel to each other and deliver energy associated with the pristine Bragg peaks outside of the patient.

### Optional examples

In an optional example, the threshold distance may be configured to ensure that the energy delivered to the PTV or OARs of the patient by any given spot path of the field interacts with energy delivered by other spot paths of the field so as to cause a cumulative amount of energy satisfying beam configuration constraints for that field to be delivered to specific portions of the patient.
In an optional example, one or more beam configurations may be determined such that each spot path of the beam configuration is configured to deliver energy to the patient for a given field without interacting with energy delivered by other spot paths of the beam configuration.
In an optional example, one or more beam configurations may be determined such that a build-up region (e.g., a region along the dose distribution curve that is located before the pristine Bragg peak or the SOBP) of each spot path is configured to intersect with a PTV.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates a plurality of beams targeting a PTV of a patient as viewed from a two-dimensional (2D) transverse view extending along an axial plane positioned relative to the patient.
**FIG. 2** illustrates a diagram of a system for configuring a proton beam system to deliver energy to a patient, according to an embodiment.
**FIG. 3** illustrates a flow diagram of a process for configuring a proton beam system to deliver energy to a patient, according to an embodiment.
**FIG. 4A** illustrates an example dose delivered to a patient using a proton beam system, according to an embodiment.
**FIG. 4B** illustrates a pattern and corresponding amounts of energy delivered to a planning target volume, according to an embodiment.
**FIG. 4C** is an example of a scanning motion that can be implemented by a medical device, according to an embodiment.
**FIG. 5A** illustrates an example dose delivered to a patient using a proton beam system, according to an embodiment.
**FIG. 5B** illustrates patterns for configuring the delivery of energy to a planning target volume, according to an embodiment.
**FIG. 5C** is an example of a scanning motion that can be implemented by a medical device, according to an embodiment.
**FIG. 6A** illustrates an example of dose distribution at distances relative to a proton beam system, according to an embodiment.
**FIG. 6B** illustrates examples of doses delivered to a patient using a proton beam system in different configurations, according to an embodiment.
**FIG. 7** illustrates an example set of fields for delivering energy to a patient, according to an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are configured to be considered within the scope of the subject matter disclosed herein. Other embodiments can be used or other changes can be made without departing from the scope of the present disclosure. The illustrative embodiments described in the detailed description are not meant to be limiting of the subject matter presented.

The systems and methods described, as well as the techniques they implement, improve conventional RT treatment planning techniques. More specifically, in embodiments, systems described herein can be implemented to optimize dose delivery when configuring a proton beam system to deliver energy to target a planning target volume (PTV) of a patient. Such systems can include one or more processors that are configured to receive scan data associated with a three-dimensional scan of a patient. The three-dimensional scan can represent at least a portion of a planning target volume. The one or more processors can determine a treatment plan involving the delivery of energy from a plurality of gantry positions relative to the patient. Each gantry position can correspond to a field according to which at least a portion of the energy is configured to be delivered to the patient. In some implementations, the one or more processors can determine, for each field, a beam configuration configured to target the planning target volume such that each beam of the beam configuration is separated from at least one other beam of the beam configuration by a distance that satisfies a threshold distance. In implementations, the one or more processors can then provide control data associated with the treatment plan to a proton beam system, the control data configured to control the proton beam system when executing the treatment plan.

By virtue of the implementation of the techniques described herein, proton beam systems can be controlled so as to reduce or eliminate beam overlap by parallel beams that can cause the dose delivered to a non-targeted portion of the patient (e.g., an OAR) to exceed desired amounts. For example, by spacing the beams by threshold distances as described herein for a given field, the chance of energy accumulating at one or more OARs during treatment of the patient can be reduced, and constraints related to dose distribution control when generating treatment plans can be lifted to allow for greater freedom when generating treatment plans. And by separating the beams in accordance with the techniques described herein, the overlap of Bragg peaks can be strategically managed, enhancing the therapeutic ratio by delivering high conformal doses to the PTV while preserving the functionality of nearby organs at risk. This precision can allow for improved energy delivery to the PTV, thereby improving patient outcomes.

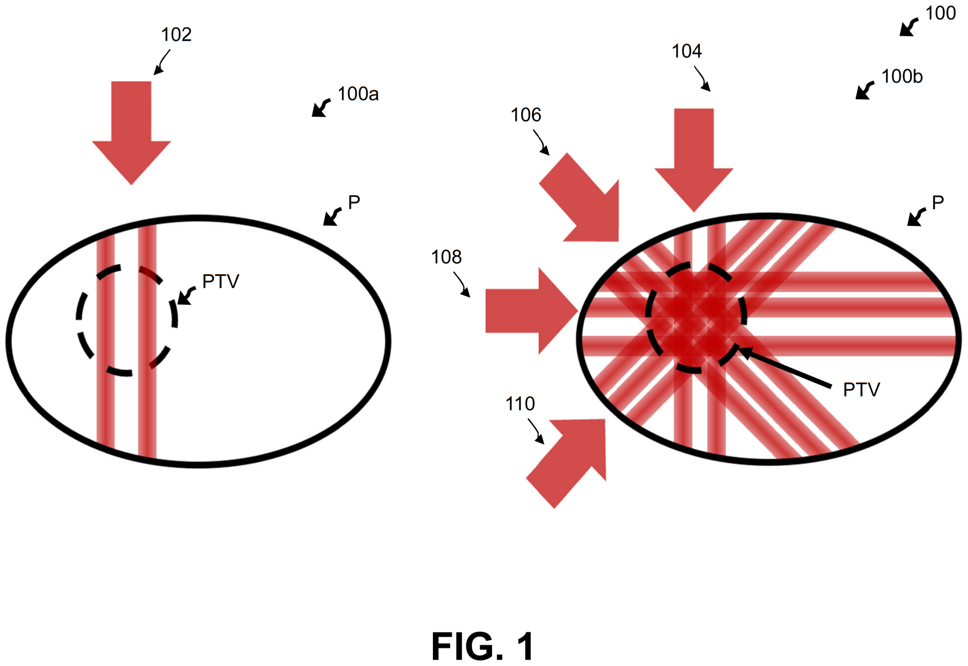

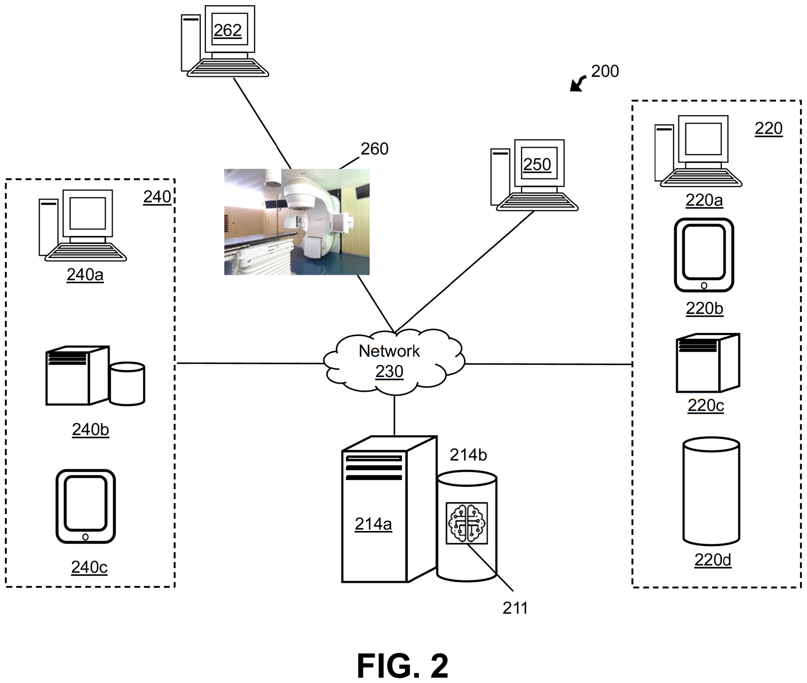

**FIG. 1** illustrates a plurality of beams targeting a PTV of a patient "P" as viewed from a two-dimensional (2D) transverse view extending along an axial plane positioned relative to the patient "P". More specifically, **FIG. 1** illustrates the delivery of energy (e.g., from a medical device that is the same as, or similar to, the medical device **260 of** **FIG. 2**) toward a PTV of a patient "P" using a plurality of beams **102-110,** where each beam is configured such that each beam transmits energy along a subset of spot paths (e.g., few sparse spots) from individual fields corresponding to each beam **102-110** to deliver dose (e.g., the amount of radiation energy delivered to a specific volume of tissue, sometimes measured in Gray (Gy)) that accumulates during a treatment session to fill a target area (including the PTV).

In a first example **100a,** the patient "P" can be targeted with a beam having a beam configuration that allows for the transmission of energy and delivery of dose by activating two spots to deliver energy along corresponding spot paths from among a plurality of possible spots within a field. In this example, as energy is transmitted from the medical device in accordance with the two spots of the field, dose is delivered to the patient "P" along two spot paths 120. By activating spots separated by a distance in accordance with the techniques described herein, dose that is delivered by respective spot paths can be isolated or minimally overlapping, allowing for reduction in the delivery of cumulative dose outside the PTV in undesired locations of the patient (e.g., OARs, etc.). In a second example **100b,** multiple beams **104-110** are illustrated as targeting the PTV of the patient "P". In this example **100b,** a gantry of the medical device can be moved to various positions/poses relative to the patient "P" and, upon activation, dose can be delivered to the patient "P" to target the PTV.

By delivering dose in accordance with the techniques described herein, the dose targeting a particular voxel (e.g., a three-dimensional pixel that represents a specific volume element of tissue within the patient's anatomy) can be delivered more quickly when compared to other proton beam therapy techniques (e.g., pencil beam scanning), allowing for the delivery of radiation at higher dose rates in less time (also referred to as FLASH radiotherapy). This rapid delivery of dose can allow for more precise targeting of tumors within the PTV of the patient "P". And by activating spots that are separated by a determined distance per field (sparse spots), the delivery of dose outside of the PTV can be minimized, reducing the affects of such dose delivery on non-target tissue.

**FIG. 2** illustrates components of a system **200** for configuring a proton beam system to deliver energy to a patient, according to an embodiment. The system **200** can include an analytics server **214a,** system database **214b,** a treatment planning system **211,** electronic data sources **220a-d** (each referred to individually as an electronic data source **220** and collectively electronic data sources **220,** unless stated otherwise), end-user devices **240a-c** (each referred to individually as an end user device **240** and collectively as end-user devices **240,** unless stated otherwise), an administrator computing device **250,** a medical device **260,** and medical device computer(s) **262.** Various components depicted in **FIG. 2** can belong to a radiotherapy clinic at which patients can receive radiotherapy treatment, in some cases via one or more radiotherapy machines (e.g., medical device **260**) located within the clinic. The system **200** is not confined to the components described herein and can include additional or other components, not shown for brevity, which are configured to be considered within the scope of the embodiments described herein.

The above-mentioned components can be connected to each other through a network **230.** Examples of the network **230** can include, but are not limited to, private or public local-area-networks (LAN), wireless LAN (WLAN) networks, metropolitan area networks (MAN), wide-area networks (WAN), and the Internet. The network **230** can include wired or wireless communications according to one or more standards or via one or more transport mediums. The communication over the network **230** can be performed in accordance with various communication protocols such as Transmission Control Protocol and Internet Protocol (TCP/IP), User Datagram Protocol (UDP), and IEEE communication protocols. In one example, the network **230** can include wireless communications according to Bluetooth specification sets or another standard or proprietary wireless communication protocol. In another example, the network **230** can also include communications over a cellular network, including, e.g., a GSM (Global System for Mobile Communications), CDMA (Code Division Multiple Access), and EDGE (Enhanced Data for Global Evolution) network.

The analytics server **214a** can be any computing device comprising a processor and non-transitory machine-readable storage capable of executing the various tasks and processes described herein. The analytics server **214a** can employ various processors such as central processing units (CPU) and graphics processing unit (GPU), among others. Non-limiting examples of such computing devices can include workstation computers, laptop computers, server computers, and the like. While the system **200** includes a single analytics server **214a,** the analytics server **214a** can include any number of computing devices operating in a distributed computing environment, such as a cloud environment.

The analytics server **214a** can generate and display an electronic platform configured to use a treatment planning system **211** for receiving patient information and inputs from users (e.g., clinicians) such as utility functions and updated utility functions described herein, and outputting the results of execution of the treatment planning system **211.** The electronic platform can include graphical user interfaces (GUI) displayed by display devices of one or more electronic data sources **220,** the end-user devices **240,** the medical device **260,** or the administrator computing device **250.** An example of the electronic platform generated and hosted by the analytics server **214a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the treatment planning system **211.** For instance, the analytics server **214a** can execute the treatment planning system **211** (e.g., a system such as a treatment planner that is configured or trained to generate beam configurations that are usable to configure the medical device **260** when treating a patient, as described herein). The analytics server **214a** can then display the results for a clinician or directly revise one or more operational attributes of the medical device **260.**

The electronic data sources **220** can be any computing device comprising a processor and non-transitory machine-readable storage capable of executing the various tasks and processes described herein. For example, the electronic data sources **220** can represent various computing devices that contain, retrieve, or access data associated with a medical device **260,** such as data associated with operational information of currently or previously performed radiotherapy treatments (e.g., electronic log files or electronic configuration files), data associated with current or previously monitored patients (e.g., computed tomography (CT) scans, magnetic resonance imaging (MRI) scans, tumor locations, deformation information, or the like) or participants in a study, or the like. For instance, the analytics server **214a** can use the clinic computer **220a,** medical professional device **220b,** server **220c** (associated with a clinician or a clinic), and database **220d** (associated with the clinician or the clinic) to retrieve/receive data associated with the medical device **260.** The analytics server **214a** can retrieve the data from the end-user devices **240,** generate a dataset, and use the dataset to configure the treatment planning system **211** (e.g., models implemented by the treatment planning system **211** or the like). The analytics server **214a** can execute various algorithms to translate raw data received/retrieved from the electronic data sources **220** into machine-readable objects that can be stored and processed by other analytical processes as described herein.

End-user devices **240** can be any computing device comprising a processor and a non-transitory machine-readable storage medium capable of performing the various tasks and processes described herein. Non-limiting examples of an end-user device **240** can be a workstation computer, laptop computer, tablet computer, or server computer. In operation, various users such as clinicians as described herein can use end-user devices **240** to access the GUI operationally managed by the analytics server **214a** or otherwise the results of the execution of the treatment planning system **211.** Specifically, the end-user devices **240** can include clinic computer **240a,** clinic server **240b,** and a medical professional device **240c.** Even though referred to herein as "end-user" devices, these devices cannot always be operated by end-users. For instance, the clinic server **240b** cannot be directly used by an end user. However, the results stored on the clinic server **240b** can be used to populate various GUIs accessed by an end user via the medical professional device **240c.** In some embodiments, the end-user device **240** can be associated with one or more clinicians that are associated with the generation of one or more treatment plans (e.g., involved in preparing the one or more treatment plans) for patients.

The administrator computing device **250** can represent a computing device operated by a system administrator. The administrator computing device **250** can be configured to display radiotherapy treatment attributes generated by the analytics server **214a** (e.g., various analytic metrics determined during training of one or more machine learning models or systems); monitor various treatment planning systems **211** utilized by the analytics server **214a,** electronic data sources **220,** or end-user devices **240;** review feedback; or facilitate training or retraining (calibration) of the treatment planning system **211** that are maintained by the analytics server **214a.**

In some embodiments, the medical device **260** can be a diagnostic imaging device or a treatment delivery device. For example, the medical device **260** can include one or more computed tomography (CT) scanners such as a cone-beam CT (CBCT) scanner, proton beam therapy systems (referred to as proton beam systems) that uses accelerated protons to deliver precise radiation therapy to tumors, or other similar devices configured to transmit energy toward targeted tissue (referred to as planning target volumes) associated with a patient and, in some cases, measure the energy transferred to ward the targeted tissue. The medical device **260** can also include one or more sensors configured to monitor the patient being treated. That is, the medical device **260** or the analytics server **214a** can be communicating with various sensors that can monitor a patient's external biological signals. Non-limiting examples of the sensors can include 3D surfacing mechanisms and optical (or other) sensors configured to monitor the patient's movements (e.g., how the patient is moving or breathing). In some embodiments, the medical device **260** can receive data associated with a treatment plan from the medical device computer(s) **262** that cause the medical device **260** to operate in accordance with the treatment plan.

The treatment planning system **211** can be stored in the system database **214b.** The treatment planning system **211** can be trained using data received/retrieved from the electronic data sources **220** and can be executed using data received from the end-user devices, the medical device **260,** or the sensor **263.** In some embodiments, the treatment planning system **211** can reside within a data repository local or specific to a clinic. In various embodiments, the treatment planning system **211** can use one or more deep learning engines to develop a treatment plan for a patient using radiation therapy. For instance, the analytics server **214a** can transmit patient attributes from the sensor **263** and execute the treatment planning system **211** accordingly. The analytics server **214a** can then display the results on one or more end-user devices **240.** In some embodiments, the analytics server **214a** can change one or more configurations of the medical device **260** based on the results predicted by the treatment planning system **211.**

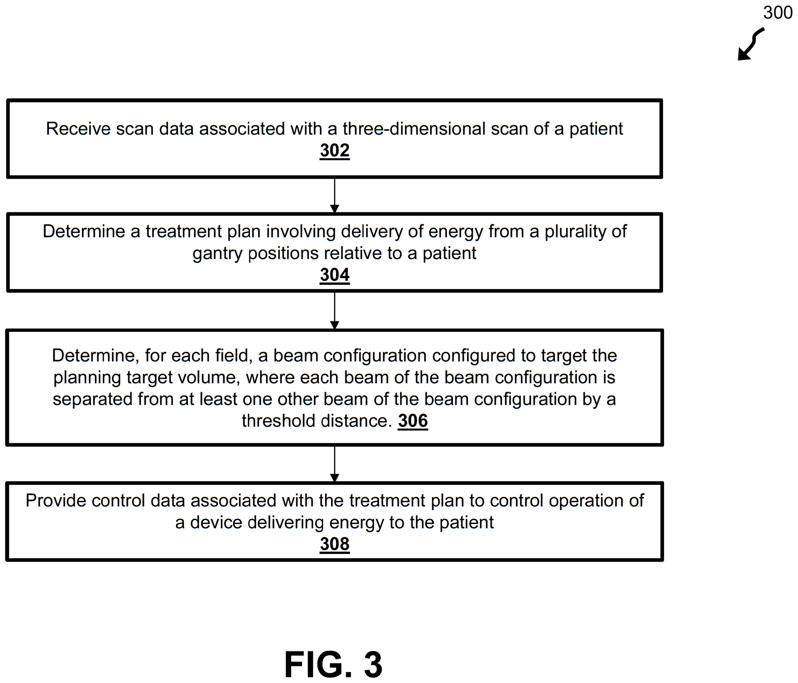

Referring to **FIG. 3****,** illustrated is a flow diagram of a process **300** for generating and evaluating radiation therapy treatment plans. The process **300** includes operations **302-308.** However, other embodiments can include additional or alternative operations or can omit one or more operations altogether. The process **300** is described as being executed by an analytics server, which can be the same as, or similar to, the analytics server **214a** described in **FIG. 2****.** However, one or more steps of the process **300** can be executed by any number of computing devices operating in the distributed computing system described in **FIG. 2****.** For instance, one or more computing devices can locally perform part or all of the operations described in **FIG. 3****.**

At operation **302,** the analytics server can receive scan data associated with a three-dimensional scan of a patient. For example, the analytics server can obtain scan data generated by a medical imaging device (e.g., a computed tomography (CT) scanner, a magnetic resonance imaging (MRI) scanner, a positron emission tomography (PET) scanner, a cone-beam computed tomography (CBCT) scanner, and/or the like) representing at least a portion of a patient. In this example, the 3D scan can represent a PTV such as a tumor or other area where the delivery of energy (e.g., using a proton beam system as described herein) is desired to treat the patient. The 3D scan can also represent one or more anatomical features that are different from the PTV. For example, the 3D scan can represent one or more organs in proximity to the PTV that can receive energy during delivery of energy by the proton beam system to the PTV but are not associated with the PTV. In this example, the one or more organs are referred to as organs at risk (OARs). It will be understood that the treatment objective of a given treatment plan can be to maximize the amount of energy delivered to a PTV while minimizing the amount of energy delivered to the OARs.

At operation **304,** the analytics server can determine a treatment plan involving delivery of energy by a medical device (e.g., a medical device that is the same as, or similar to, the medical device **260 of** **FIG. 2**) from a plurality of gantry positions relative to a patient. For example, the analytics server can determine a treatment plan involving delivery of energy from a plurality of gantry positions at which one or more proton beams are transmitted at varying power levels. These proton beams can be configured to target portions of the body (e.g., PTVs or the like) as described herein. In some embodiments, each gantry position can be associated with a control point. For example, each gantry position can be associated with a control point at which the gantry of the medical device is configured to move when generating proton beams for a given field to target one or more PTVs. Each control point can be represented as a set of parameters or configurations at which the medical device adjusts or changes during the beam delivery process.

In some embodiments, the analytics server can determine a treatment plan such that the treatment plan specifies a plurality of proton spot paths to be generated at one or more fields to target a PTV and deliver energy to the PTV while minimizing exposure to healthy tissue (including OARs). For example, the analytics server can determine a treatment plan based on the location, size, shape, or the like of the PTV. In this example, the analytics server can determine the treatment plan based on the analytics server determining the PTV (e.g., a location of the PTV, extents of an exterior surface of the PTV, or the like) based on the three-dimensional scan of the patient. The analytics server can then translate the data associated with the PTV into specific angles or positions from which the gantry will deliver the proton beams. As described herein, each gantry position can be determined to ensure that the proton beams' Bragg peaks (e.g., where protons deposit most of their energy) are aligned precisely with different segments or layers of the PTV. This multi-angle approach can allow for a highly conformal dose distribution, sculpting the radiation closely around the tumor, thereby enhancing the effectiveness of the treatment while preserving surrounding critical structures.

In some embodiments, the analytics server can determine one or more aspects of the treatment plan based on one or more treatment objectives. These treatment objectives can represent one or more desired aspects of delivery of energy of the patient. For example, treatment objectives can include any combination of one or more of a target coverage (e.g., a volume of the PTV to receive a prescribed amount of radiation as compared to the entire PTV), a tumor eradication rate (e.g., a probability that all of the cancer cells in the PTV will be eliminated), satisfaction of an amount of energy delivered to a PTV (e.g., as measured by an amount of energy delivered to the entire PTV), satisfaction of a maximum amount of energy delivered to an OAR (e.g., as measured by an amount of energy delivered to the OAR), conformance of energy delivery to the PTV (e.g., an amount of the PTV receiving energy delivered at a given rate as compared to the total volume of the PTV), establishing homogeneity of energy delivery to the PTV (e.g., based on determination of a homogeneity index), or the like. The at least one treatment objective can be determined based on input by a clinician specifying the at least one treatment objective. For example, a treatment objective associated with delivery of energy to OARs can be specified by a clinician based on the clinician providing input (e.g., at an end user device that is the same as, or similar to, the end user devices **240 of** **FIG. 2**) indicating that the relative volume of the OAR where more than 50 Gy energy was delivered should not exceed 10% and that candidate treatment plans should not be accepted when the aforementioned relative volume exceeds 20%.

At operation **306,** the analytics server can determine, for each field, a beam configuration to target a PTV. For example, the analytics server can determine a beam configuration for each field according to which the medical device will deliver energy to the patient, where each spot path of a given beam configuration runs along a spot path (e.g., from a cyclotron through the patient) parallel (with slight divergence from a fixed point source) with the other spot paths running along respective spot paths of that field. In the examples described herein, the analytics server can determine each beam configuration such that a set of spot paths of each beam configuration have dose distribution curves that form pristine Bragg peaks, spread-out Bragg peaks (SOBPs), or combinations thereof. For example, the analytics server can determine each beam configuration such that one or more spot paths of the beam configuration have dose distribution curves that form pristine Bragg peaks or and are configured to be transmitted by the medical device to target a PTV of the patient.

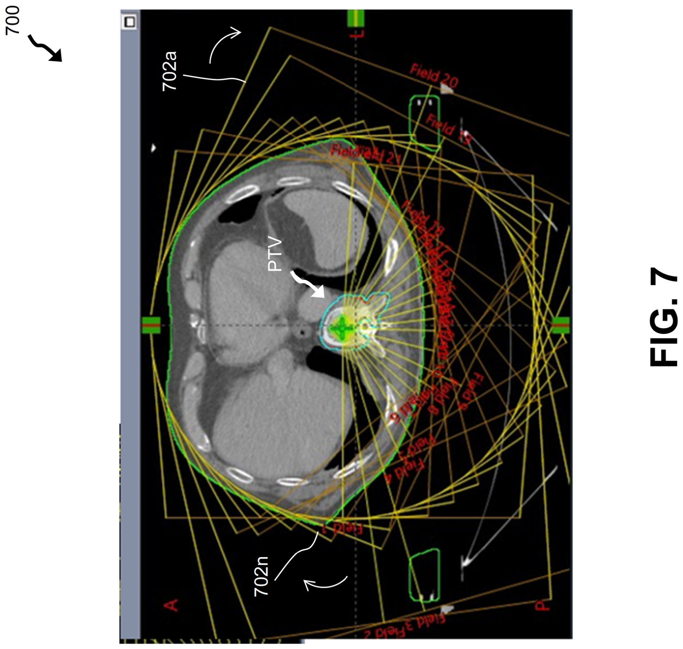

In some examples, the analytics server can determine the beam configurations such that each beam configuration of a treatment plan satisfies one or more beam configuration constraints. In some embodiments, the beam configuration constraints can be based on one or more aspects associated with the spot paths to be transmitted and/or one or more treatment objectives. For example, the beam configuration constraints for a given field can be based on one or more spot paths transmitted by one or more prior fields toward the PTV, one or more spot paths to be transmitted in a current field toward the PTV, or one or more spot paths to be transmitted in future fields toward the PTV. Examples of fields that can be used to target a PTV are illustrated as fields **702a-702n in** **FIG. 7****.** Additionally, or alternatively, the beam configuration constraints for a given field can be based on energy delivered or energy to be delivered at various points at one or more prior fields, the current fields, or at one or more future fields, within the patient. This can include the instant or cumulative energy delivered or to be delivered to a PTV, one or more OARs in the spot path targeting the PTV, and/or the like. In some embodiments, the analytics server can determine the beam configuration constraints for a given field based on one or more aspects representing a state of the patient. Such aspects can include a location associated with the PTV relative to the patient and the field, a size of the PTV, the location of OARs relative to the PTV and the spot path as the spot path passes through the patient, an amount of energy absorbed or to be absorbed at various locations within the patient including the PTV or OARs, the types of PTVs or OARs (e.g., lung, kidney, liver, etc.) targeted by the spot path, and/or the like.

In some embodiments, the analytics server can determine the beam configuration for a given field such that the spot paths of the given field are separated from at least one other spot path of the beam configuration by a threshold distance, for example a non-zero threshold distance. For example, the analytics server can determine the beam configuration for a given field such that each spot path of the field is separated by the threshold distance. The analytics server can determine the threshold distance for each field such that the threshold distance satisfies the beam configuration constraints for that field. In some examples, the threshold distance can be configured to ensure that the energy delivered to the PTV or OARs of the patient by any given spot path of the field does not interact with energy delivered by other spot paths of the field. In some examples, the threshold distance can be configured to ensure that the energy delivered to the PTV or OARs of the patient by any given spot path of the field interact with energy delivered by other spot paths of the field so as to cause a cumulative amount of energy satisfying the beam configuration constraints for that field to be delivered to specific portions of the patient. By determining beam configurations where the spot paths of a given field are separated by at least a threshold distance that satisfies the beam configuration constraints, the analytics server can develop treatment plans that reduce or eliminate the chance for energy from multiple spot paths to aggregate in OARs or other non-targeted areas, or expand beyond a surface established by the PTV, and cause a significant increase in the aggregated amount of energy delivered to OARs of the patient beyond the PTV.

In some embodiments, the analytics server can determine the spot paths forming the beam configuration for a given field such that one or more regions of the dose distribution curve corresponding to the spot paths align (e.g., intersect) with one or more areas (e.g., PTVs or OARs) relative to the patient. For example, the analytics server can determine a set of spot paths (e.g., a subset of all of the spot paths that can be transmitted for a given field) to be generated by the medical device when treating the patient. In this example, each spot path of the set of spot paths can be spaced such that the spot paths are separated by at least a threshold distance to satisfy one or more beam configuration constraints as described herein.

In one example, the analytics server can determine one or more beam configurations such that each spot path of the beam configuration is configured to deliver energy to the patient for a given field without interacting with energy delivered by other spot paths of the beam configuration. For example, in comparison with pencil beam scanning techniques where spot paths are moved across a field (e.g., activated) from position to position in a continuous sequence, the analytics server can determine one or more beam configurations where a specific subset of spot paths that can be generated in a given field are formed. The analytics server can determine the beam configuration such that each spot path of the subset of spot paths are spaced in accordance with the threshold distance to prevent the energy from each spot path interacting with energy from other spot paths when delivered to the patient and causing a buildup of energy at any point within the patient along the spot paths to exceed an amount that is permitted by the beam configuration constraints.

In another example, the analytics server can determine one or more beam configurations such that a build-up region (e.g., a region along the dose distribution curve that is located before the pristine Bragg peak or the SOBP) of each spot path is configured to intersect with (e.g., align with) a PTV. In this example, the analytics server can determine that a different region beyond the build-up region (e.g., a Bragg peak region along the dose distribution curve that is associated with the formation of the pristine Bragg peak) intersects with a portion of the patient along the spot path that is beyond the PTV and that an amount of cumulative energy delivered to the region beyond the build-up region satisfies a threshold amount (e.g., energy delivered by each of the spot paths for a given field is aggregated in the region such that the energy is equal to or less than a predetermined amount of energy). In this way, the analytics server can determine a spacing for a given spot path configuration between the spot paths such that a desired amount of energy is delivered to the PTV while constraining the cumulative amount of energy delivered to regions beyond the PTV, for example to an OAR.

In another example, the analytics server can determine a beam configuration such that a different region beyond the build-up region of a given spot path does not intersect with a portion of the patient (e.g., along a portion of the spot path that has exited the patient). For example, the analytics server can determine a beam configuration such that a build up region associated with the dose distribution curve of the spot paths intersects with the PTV of the patient and the Bragg peak region is formed outside of the patient. This, in turn, can allow the analytics server to determine beam configurations such that doses of energy can be more consistently delivered to the patient across larger regions of the PTV of the patient without error in alignment that can be associated with the targeting of the PTV with spot path forming a Bragg peak.

When determining the beam configurations for a treatment plan, the analytics server can assign a plurality of spot paths to a field. For example, the analytics server can assign a plurality of spot paths having desired power levels to positions within a field such that a desired amount of energy is delivered by each spot path to target the PTV. In one example, the analytics server can assign the plurality of spot paths so as to reduce or eliminate the probability that energy delivered to the patient by the plurality of spot paths overlaps. In this example, the analytics server can forgo assigning other spot paths to a beam configuration for a given field where it is expected that the assignment of such spot paths will result in energy delivery within the patient that overlaps and, as a result, does not satisfy the beam configuration constraints for that field. In another example, the analytics server can assign a plurality of spot paths to a beam configuration of a field such that the spot paths of the beam configuration do not overlap with energy delivered to the patient by each other spot path in a specific region of the patient. For example, the analytics server can assign a plurality of spot paths to a beam configuration of a field such that the spot paths do not deliver energy that overlaps with one another at an area that is outside of a threshold distance that is associated with the PTV.

In some embodiments, the analytics server can assign a plurality of spot paths to a beam configuration of a field such that energy delivered to the patient by each spot path targets the PTV. Once assigned, the analytics server can determine a set of spot paths from the plurality of spot paths of the field that are configured to deliver energy to a location that is outside of a threshold distance that is associated with the PTV. The location can include, for example, a predetermined OAR that is located before or after the PTV along an axis defined by the spot paths of the field and will receive energy from the spot paths of a given field. In some examples, the analytics server can then unassign a subset of spot paths included in the set of spot paths such that energy delivered by the plurality of spot paths does not overlap at the location outside of the threshold distance. In some examples, the analytics server can then unassign a subset of spot paths included in the set of spot paths from the plurality of spot paths such that energy delivered by the plurality of spot paths does not result in energy delivered beyond a threshold amount at the location outside of the threshold distance.

At operation **308,** the analytics server can provide control data associated with the treatment plan to control the operation of a device delivering energy to the patient. For example, the analytics server can provide control data associated with the treatment plan during one or more phases of treatment of the patient. In an example, the analytics server can provide the control data to a medical device computer (e.g., a medical device computer that is the same as, or similar to, the medical device computer(s) **262** of **FIG. 2**) that is configured to control operation of a medical device (e.g., a medical device that is the same as, or similar to, the medical device **260** of **FIG. 2**). The control data can be configured to cause the medical device to perform one or more operations. For example, where the medical device includes a proton beam system, the control data can be configured to cause a gantry of the proton beam system to move such that a cyclotron is positioned to deliver energy in accordance with one or more beam configurations for one or more fields. In this way, the control data can be configured to control the medical device (e.g., the proton beam system) when executing the treatment plan.

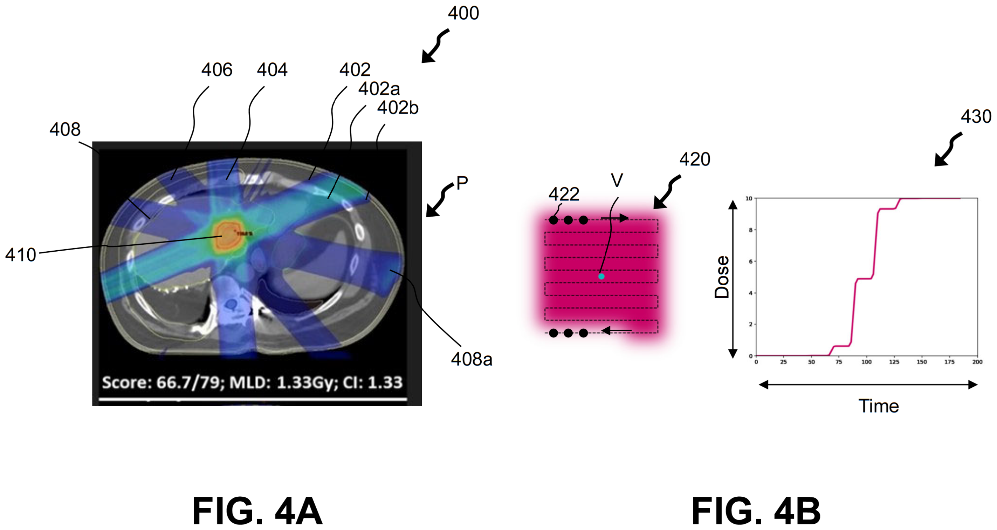

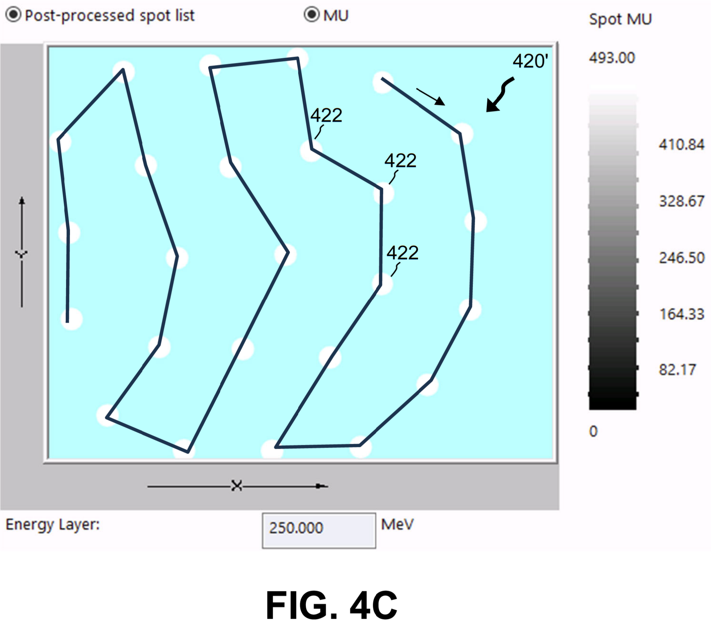

Referring now to **FIGS. 4A** and **4B, FIG. 4A** illustrates an example dose delivered to a patient using a medical device such as a proton beam system, according to an embodiment, and **FIG. 4B** illustrates a pattern according to which energy can be transmitted and corresponding amounts of dose delivered to a planning target volume, according to an embodiment. As shown in **FIG. 4A****,** beams **402-408** including sets of proton spot paths (referred to as "spot paths **420**") can be generated by a medical device (e.g., a medical device that is the same as, or similar to, the medical device **260** of **FIG. 2**) to target a region **410** that is associated with a PTV. The spot paths **420** of each beam **402-408** can correspond to dose delivered therealong within a patient "P" so as to target dose delivery to the region **410** while minimizing dose delivered outside of the region **410.** To deliver dose to the region **410,** the medical device can be configured to generate proton beams within a given field to target the region **410** such that each spot path **420** of a given field is generated in sequence based on transmission of energy along a plurality of spot paths **420.** In this example, the back-and-forth motion as the medical device activates successive spots to transmit energy along the spot paths **422** can define a pattern according to which a voxel **"V"** within the region **410** is targeted. As a result, dose can be delivered to the region **410** such that the voxel **"V"** is targeted and receives the dose in accordance with the dose delivery graph **430** of **FIG. 4B****.** This graph shows that the time dose is actively being deposited to a voxel "V" when the plot is increasing in energy along on y-axis. When the plot is flat, no dose is being deposited, yet time used to determine the dose rate continues, reducing the calculated dose-rate. This "dead time" is due to the back-and-forth sweeping or scanning motion where, for certain periods of time, dose is being delivered around, but not to, the voxel **"V".** An alternative example of such a scanning motion can be seen in **FIG 4C****.**

With continued reference to **FIG. 4A****,** by virtue of the spacing of the beams relative to the patient and corresponding spacing of spots that are activated within the field, dose can be delivered that is aggregated and accumulates (e.g., compounds) as spot paths **422** are spaced closer together for a given field outside of the region **410.** For example, delivery of dose or energy along the plurality of proton spot paths **402** can result in multiple sub-regions **402a, 402b,** within the patient where energy accumulates by virtue of the spacing of the beams and the power level at which the beams are transmitted. As a result, sub-regions associated with a center of a given field (e.g., sub-region **402a** of proton spot path **402**) can have higher energy delivery outside of the region **410,** and other sub-regions (e.g., sub-region **402b** of proton spot path **402**) can have lower energy delivery outside of the region **410,** for example, by virtue of the beams of the other sub-regions being located adjacent to fewer beams (e.g., when located along a periphery of the proton spot path **402**)**.** In another example, delivery of energy along the proton spot path **408** at certain areas beyond the region **410** can result in sub-regions such as sub-region **408a** having higher energy delivery in the sub-region **408a** and lower energy delivery along the proton spot path **408** outside of the sub-region **408a,** for example, by virtue of the formation of pristine Bragg peaks in the sub-region **408a.**

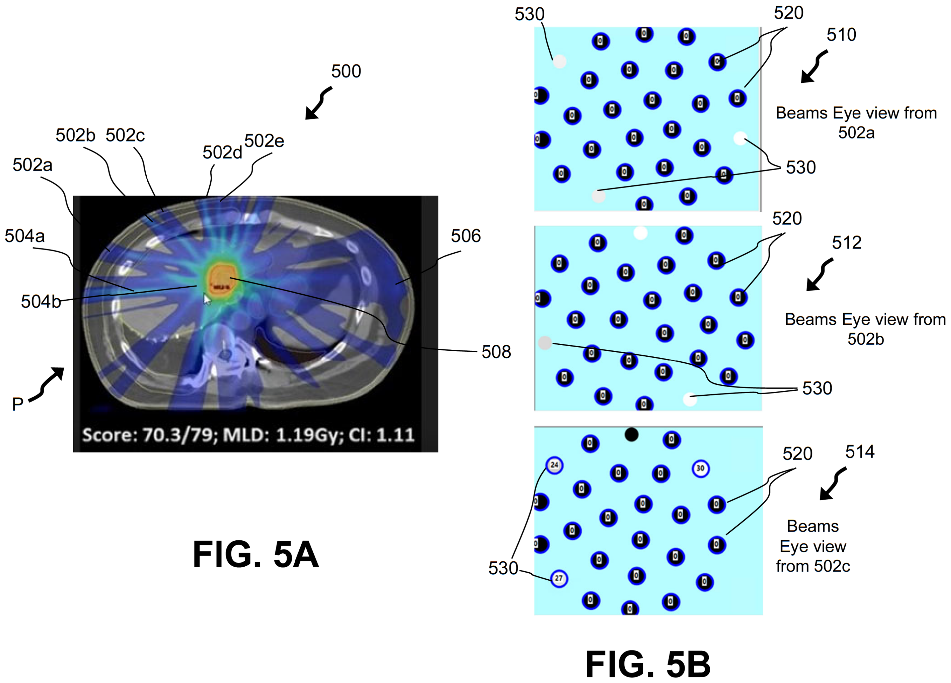

Referring now to **FIGS. 5A** and **5B****,** illustrated is an example dose delivered to a patient using a medical device such as a proton beam system, according to an embodiment, and patterns for configuring transmission of energy and delivery of dose to a PTV, according to an embodiment. As shown in **FIG. 5A****,** multiple sets of proton beam paths **502a-502e** (collectively referred to as proton beam paths **502** for purposes of clarity) can be generated by a medical device (e.g., a medical device that is the same as, or similar to, the medical device **260** of **FIG. 2**) to target a region **508** that is associated with a PTV. Each proton beam path **502** can include one or more spot paths **520** along which energy can be (or can selectively not be) transmitted toward the patient "P", and dose is delivered within the patient "P" so as to target dose delivery to the region **508** while optimizing one or more treatment objectives as described herein. To deliver dose to the region **508,** the medical device can be configured to generate proton beams in accordance with a corresponding field to target the region **508** such that each spot path of each proton beam for the field is generated in accordance with one or more beam configurations as described herein. Examples of beam configurations that can satisfy one or more beam configuration constraints as described herein are illustrated in **FIG. 5B****.**

In some embodiments, the beam configurations of **FIG. 5B** can identify one or spot paths **520** included in a beam path **502 of** **FIG. 5A****.** For example, when generating a first proton beam path **502a,** an analytics server (e.g., that is the same as, or similar to, the analytics server **214a** of **FIG. 2** and/or the analytics server described with respect to **FIG. 3**) can determine beam configurations such that energy delivery outside of the region **508** is minimized. For example, when delivering energy in accordance with a first beam path **502a,** the medical device can cause energy to be directed toward the patient "P" along one or more spot paths **530** in accordance with a first beam configuration **510** of **FIG. 5B****.** In this first beam configuration **510,** the analytics server can configure the medical device to deactivate (e.g., not transmit) energy for one or more spot paths **520** to allow one or more other spot paths **530** to satisfy the beam configuration constraints for the corresponding field. This process can be iteratively repeated across multiple fields when generating a treatment plan for a patient in accordance with one or more treatment objectives. As a result, as illustrated by the dose distributed to the patient in **FIG. 5A****,** by not activating certain spot paths **520** for a given beam path **502** in accordance with the beam configurations **510-514,** dose can be delivered along the spot paths **530** to minimize the amount of aggregated energy being delivered to areas outside of the region **508** being targeted for treatment. In another example, delivery of energy along the beam paths **502** at certain areas beyond the region **508,** such as sub-region **506,** can have relatively lower amounts of dose delivered where pristine Bragg peaks are formed as a result of no adjacent spot paths accumulating from expanding Bragg peaks nearby. This can reduce or, in some cases eliminate, the effects of having dose delivered to OARs on the patient "P" while still allowing for a sufficient amount of dose to be delivered to satisfy the treatment objectives for the treatment plan.

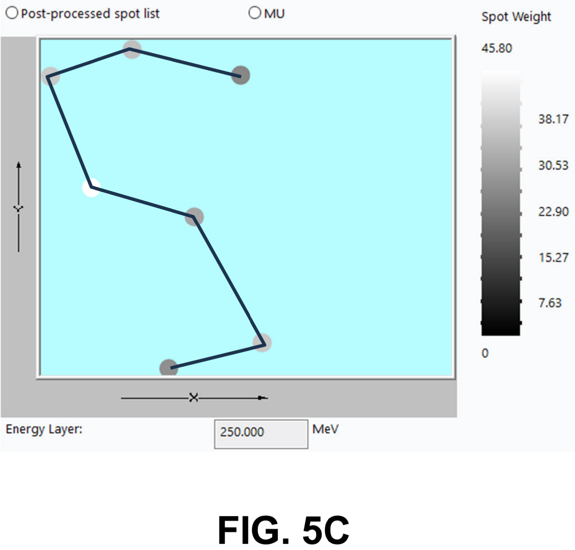

In some embodiments, multiple beam configurations of **FIG. 5B** can be used to generate one or more of the proton spot paths **502.** For example, the analytics server can configure the medical device to generate energy in accordance with multiple beam configurations **510-514** for a given unique beam gantry orientation **502a.** In one example, the analytics server can configure the medical device to deliver the first beam configuration **510,** from a first orientation **502a** the second beam configuration **502b** from a second orientation **512,** and the third beam configuration **514** from the third orientation **502c** such that the spot paths **530** that are activated for a given beam configuration rotate relative about the patient to create each field to establish a given spot path **502.** In this example, the spot paths **530** can be activated such that they form a pattern (e.g., a star pattern or a cross pattern) that allows for more even distribution of dose to the patient "P". By delivering dose in accordance with this pattern, a uniform distribution can be achieved. Compared to other techniques this method allows for more beam orientations with less active spot paths in each orientation. This results, in few or no voxel re-irradiations within a single beam orientation and effectively no back-and-forth scanning and no "dead time" thus faster calculated dose rates (e.g., scanning techniques illustrated by **FIGS. 4B** **and** **4C**). An example beams eye view of a beam orientation with less unique beam paths can be seen in **FIG. 5C****.** Such a sparse arrangement of beam paths can result in a snake pattern that no longer returns to any nearby voxels (no "dead time") as seen in **FIG. 5C****.** This reduced number of spot paths can allow the analytics server the flexibility of strategically placing each minimizing the effects of dose delivered by beams that overlap. This, in turn, can allow for more continuous and consistent delivery of dose to the region **508** of the patient "P" without increasing the amount of energy delivered outside of the region **508.**

By generating spot paths in accordance with multiple beam configurations that can target a PTV from multiple, unique gantry position, fields can be selected and configured to target the PTV with fewer spots per field than conventional treatment techniques. As a result, the spots per field can be configured such that tissue is not irradiated twice within the same field (e.g., the spot never "scans" back to trigger a re-irradiation). Furthermore, inter-field overlaps in dose delivered outside the target can be minimized by precise positioning of each spot path in global all-field-simultaneous-spot-position-optimization algorithms. Examples of such algorithms are identified in U.S. Pat. Appl. Pub. No. 2024/0042236 to Lansonneur, titled "RADIOTHERAPY DOSE PARAMETER COMPUTATION TECHNIQUES" the contents of which are hereby incorporated by reference in their entirety and for all purposes. As a result, overlaps are managed at the per-spot path level as opposed to the per-field level, and each individual field can be configured to deliver a very heterogeneous dose distribution when targeting the PTV of the patient. In some embodiments, beam configurations can be arranged such that the spot paths of the beams formed for a given beam configuration interdigitate to create a summative homogeneous dose in the PTV or arranged to maintain a heterogeneous summative target dose distribution. In some embodiments, a given beam configuration can configure a medical device to transmit beams for one or more frames at the highest energy level the system supports and with the nozzle current at maximum. This can result in FLASH plans with very high dose rates independent of field or target size (as the pencil beam scanning (PBS) dose rate is now equal to the instantaneous dose rate). In one example, a 20 field plan could be treated in ~30 seconds on a FLASH system and ~90 seconds on a standard proton beam system such as the Varian^{®} ProBeam^{®} system. Further, by configuring the proton beam system to delivery energy at the maximum amount at which the system is configured to transmit, the Bragg peak can be configured to occur outside the target and, where achievable, outside the patient. And due to the absence of lateral energy scatter (by virtue of the spot paths being sparse enough to prevent or minimize such energy scatter) the Bragg peak from an individual spot can be configured to be far less intense than would otherwise be the case when scanning a field.

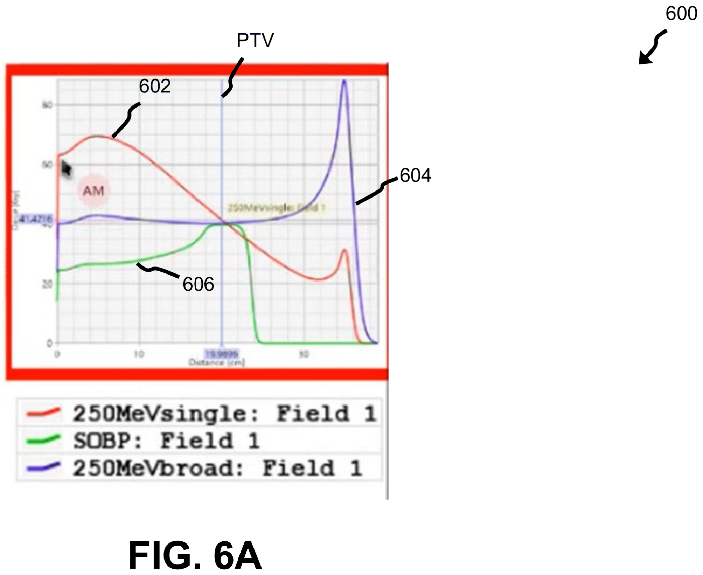

**FIG. 6A** illustrates an example of dose distribution at distances relative to a medical device (e.g., that is the same as, or similar to, the medical device **260** of **FIG. 2**) such as a proton beam system, known as a percent depth dose (PDD) curve, according to an embodiment. As shown in **FIG. 6A****,** proton beams can be configured to target a PTV such that the spot paths of the proton beams first enter the periphery of the patient at a first distance (e.g., 0 cm), interact with the tissue of the patient (e.g., OARs) as the spot path traverses the body of the patient (e.g., from 0 cm to approx. 18 cm), interact with the tissue of the PTV (e.g., from approx. 18 cm to 22 cm), and then interact with additional tissue of the patient (e.g., from approx. 22 cm to 30 cm) until potentially exiting the patient. In one example, an isolated single spot path **602** is transmitted such that higher levels of absorption are observed in a first region before interacting with the PTV (e.g., between 0-10 cm), and a relatively small Bragg peak effect is formed in a second region after the PTV (e.g., between 34-36 cm). In another example, a second broad proton beam consisting of many spots paths in close proximity **604** is transmitted at a power level that matches that of **602,** but in broad beam form, creates a much larger a pristine Bragg peak in the distant region (e.g., between 32-38 cm). In yet another example, a third proton beam, again broad, **606** is transmitted at multiple lower energy levels compared to the first proton beam **602** and the second proton beam **604** such that the largest dose is deposited at the PTV region by stacking several smaller Bragg Peak effects to create a so-called Spread Out Bragg Peak (SOBP) effect, (e.g., between 18-22 cm), and the dose sharply declines after exiting the PTV. By determining beam configurations that reduce the interactions between proton beams as described herein, energy can be more consistently delivered in accordance with the various dose distribution profiles illustrated by **FIG. 6A****,** allowing for more consistent delivery of dose to PTVs while minimizing undesired dose delivery outside of the PTVs (e.g., aggregation of energy from multiple beam orientations covering only portions of the PTV and avoiding OARs).

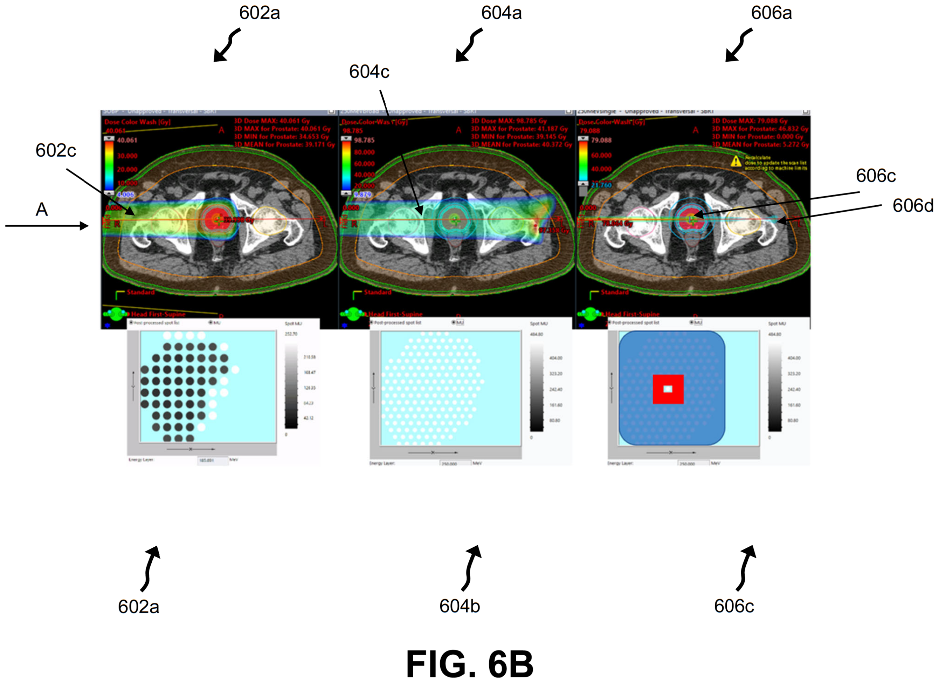

**FIG. 6B** illustrates examples of doses delivered to a patient using a proton beam system in different configurations, according to an embodiment. As shown in **FIG. 6B****,** in a first example **602a,** beam paths can target a patient according to a first beam configuration **602b** along an axis "A". In this example, the first beam configuration **602b** can include all or substantially all of the spot paths that intersect with the PTV being activated, and the dose can be delivered to a region **602c** extending through at least a portion of the patient (e.g., from where the spot paths enter the patient to the PTV). In a second example **604a,** each of the spot paths of a given field are activated. As a result, dose can be delivered to the patient according to the second beam configuration **604b** along an axis "A" such that dose is evenly distributed throughout the patient within a region **604c** extending through at least a portion of the patient, and energy aggregates where Bragg peaks are formed just before the beams exit the patient. In this example, the Bragg peaks can cause an increased delivery of energy to an OAR such for example, a kidney of the patient. In a third example **606a,** a single spot path for a beam path is activated. As a result, dose is delivered to the patient within a region **606c** and a relatively smaller region **606d** beyond the PTV receives energy as a result of the formation of a Bragg peak. However, because the dose delivered by the Bragg peak is not compounded by other Bragg peaks forming by beams traversing the patient for the field, the amount of dose delivered by the Bragg peak to the OAR is minimized in comparison to the similar region as shown in the second example **606b.**

**FIG. 7** illustrates an example set of fields **700** for delivering energy to a patient, according to an embodiment. As shown in **FIG. 7****,** a set of fields **700** can include individual fields **702a-702n** (referred to individually as a field **702** and collectively as fields **702**)**.** Each field **702** can be associated with a beam configuration identifying one or more spots corresponding to spot paths along which energy is to be transmitted. When configured as described herein, dose can be delivered to the patient via the spot paths such that dose delivered within a PTV is maximized, while dose delivered outside of a PTV (e.g., before or after the PTV along a given spot path) is minimized. As a result, higher conformance with the PTV can be obtained while an amount of time involved in delivering the dose to the PTV is reduced relative to conventional techniques for delivering dose to the PTV using medical devices as described herein.

The various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans can implement the described functionality in varying ways for each particular application, but such implementation decisions should not be interpreted as causing a departure from the scope of this disclosure or the claims.

Embodiments implemented in computer software can be implemented in software, firmware, middleware, microcode, hardware description languages, or any combination thereof. A code segment or machine-executable instructions can represent a procedure, a function, a subprogram, a program, a routine, a subroutine, a module, a software package, a class, or any combination of instructions, data structures, or program statements. A code segment can be coupled to another code segment or a hardware circuit by passing or receiving information, data, arguments, parameters, or memory contents. Information, arguments, parameters, data, etc., can be passed, forwarded, or transmitted via any suitable means, including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions can be stored as one or more instructions or code on a non-transitory computer-readable or processor-readable storage medium. The steps of a method or algorithm disclosed herein can be embodied in a processor-executable software module, which can reside on a computer-readable or processor-readable storage medium. A non-transitory computer-readable or processor-readable media includes both computer storage media and tangible storage media that facilitate the transfer of a computer program from one place to another. A non-transitory processor-readable storage media can be any available media that can be accessed by a computer. By way of example, and not limitation, such non-transitory processor-readable media can comprise RAM, ROM, EEPROM, CD-ROM or other optical disk storage, magnetic disk storage or other magnetic storage devices, or any other tangible storage medium that can be used to store desired program code in the form of instructions or data structures and that can be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm can reside as one or any combination or set of codes or instructions on a non-transitory processor-readable medium or computer-readable medium, which can be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein can be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A system for optimizing dose delivery when configuring a proton beam system to deliver energy to target a planning target volume, PTV, the system comprising:
one or more processors configured to:
receive scan data associated with a three-dimensional scan of a patient, the three-dimensional scan representing at least a portion of a planning target volume;
determine a treatment plan involving delivery of energy from a plurality of gantry positions relative to the patient, where each gantry position corresponds to a field according to which at least a portion of the energy is configured to be delivered to the patient;
determine, for each field, a beam configuration to target the planning target volume such that each spot path of the beam configuration is separated from at least one other spot path of the beam configuration by a distance that satisfies a threshold distance;
update the treatment plan based on the beam configuration for each field; and
provide control data associated with the treatment plan to a proton beam system, the control data configured to control the proton beam system when executing the treatment plan.

2. The system of claim 1, wherein the one or more processors configured to determine the beam configuration for each field are configured to:
assign each spot path of the beam configuration to a position within the field such that energy delivered to the patient by a first spot path of the beam configuration does not overlap with energy delivered to the patient by a second spot path of the beam configuration.

3. The system of claim 1 or claim 2, wherein the one or more processors configured to determine the beam configuration for each field are configured to:
assign each spot path of the beam configuration to a position within the field such that energy delivered to the patient by a first spot path of the beam configuration does not overlap with the energy delivered to the patient by a second spot path outside of a threshold distance associated with the PTV.

4. The system of any preceding claim, wherein the one or more processors configured to determine the beam configuration for each field are configured to:
assign a plurality of spot paths to the beam configuration such that energy delivered to the patient by each spot path targets the PTV;
determine a set of spot paths that are configured to deliver energy to a location outside of a threshold distance associated with the PTV; and
unassign a subset of spot paths included in the set of spot paths from the plurality of spot paths such that energy delivered by the plurality of spot paths does not overlap at the location outside of the threshold distance;
and/or:
wherein the one or more processors configured to determine the beam configuration for each field are configured to:
assign a plurality of spot paths to the beam configuration such that energy delivered to the patient by each spot path targets the PTV;
determine a set of spot paths that are configured to deliver energy to a location outside of a threshold distance associated with the PTV; and
unassign a subset of spot paths included in the set of spot paths from the plurality of spot paths such that energy delivered by the plurality of spot paths does not result in energy delivered beyond a threshold amount at the location outside of the threshold distance.

5. The system of any preceding claim, wherein the one or more processors configured to determine the beam configuration for each field are configured to:
determine the beam configuration such that a set of spot paths of the beam configuration form pristine Bragg peaks along spot paths that run parallel to each other;
and/or:
wherein the one or more processors configured to determine the beam configuration are configured to:
determine the beam configuration such that a set of spot paths of the beam configuration form pristine Bragg peaks along spot paths that run parallel to each other and deliver a cumulative amount of energy that satisfies a threshold value at a location beyond the PTV along an axis defined by the set of spot paths.

6. The system of any preceding claim, wherein the one or more processors configured to determine the beam configuration are configured to:
determine the beam configuration such that a set of spot paths of the beam configuration form pristine Bragg peaks along spot paths that run parallel to each other and deliver energy associated with the pristine Bragg peaks outside of the patient.

7. A method for optimizing a treatment plan when configuring a proton beam system to deliver energy to target a planning target volume, PTV, the method comprising:
receiving, by one or more processors, scan data associated with a three-dimensional scan of a patient, the three-dimensional scan representing at least a portion of a planning target volume;
determining, by the one or more processors, a treatment plan involving delivery of energy from a plurality of gantry positions relative to the patient, where each gantry position corresponds to a field according to which at least a portion of the energy is configured to be delivered to the patient;
determining, for each field and by the one or more processors, a beam configuration configured to target the planning target volume such that each spot path of the beam configuration is separated from at least one other spot path of the beam configuration by a distance that satisfies a threshold distance; and
updating the treatment plan based on the beam configuration for each field.

8. The method of claim 7, wherein determining the beam configuration for each field comprises:
assigning, by the one or more processors, each spot path of the beam configuration to a position within the field such that energy delivered to the patient by a first spot path of the beam configuration does not overlap with energy delivered to the patient by a second spot path of the beam configuration.

9. The method of claim 7 or claim 8, wherein determining the beam configuration for each field comprises:
assigning, by the one or more processors, each spot path of the beam configuration to a position within the field such that energy delivered to the patient by a first spot path of the beam configuration does not overlap with the energy delivered to the patient by a second spot path outside of a threshold distance associated with the PTV.

10. The method of any of claim 7 to claim 9, wherein determining the beam configuration for each field comprises:
assigning, by the one or more processors, a plurality of spot paths to the beam configuration such that energy delivered to the patient by each spot path targets the PTV;
determining, by the one or more processors, a set of spot paths that are configured to deliver energy to a location outside of a threshold distance associated with the PTV; and
unassigning, by the one or more processors, a subset of spot paths included in the set of spot paths from the plurality of spot paths such that energy delivered by the plurality of spot paths does not overlap at the location outside of the threshold distance;
and/or:
wherein determining the beam configuration for each field comprises:
assigning, by the one or more processors, a plurality of spot paths to the beam configuration such that energy delivered to the patient by each spot path targets the PTV;
determining, by the one or more processors, a set of spot paths that are configured to deliver energy to a location outside of a threshold distance associated with the PTV; and
unassigning, by the one or more processors, a subset of spot paths included in the set of spot paths from the plurality of spot paths such that energy delivered by the plurality of spot paths does not result in energy delivered beyond a threshold amount at the location outside of the threshold distance.

11. The method of any of claim 7 to claim 10, wherein determining the beam configuration for each field comprises:
determining, by the one or more processors, the beam configuration such that a set of spot paths of the beam configuration form pristine Bragg peaks along spot paths that run parallel to each other;
and/or:
wherein determining the beam configuration comprises:
determining, by the one or more processors, the beam configuration such that a set of spot paths of the beam configuration form pristine Bragg peaks along spot paths that run parallel to each other and deliver a cumulative amount of energy that satisfies a threshold value at a location beyond the PTV along an axis defined by the set of spot paths.

12. The method of any of claim 7 to claim 11, wherein determining the beam configuration comprises:
determining, by the one or more processors, the beam configuration such that a set of spot paths of the beam configuration form pristine Bragg peaks along spot paths that run parallel to each other and deliver energy associated with the pristine Bragg peaks outside of the patient.

13. A non-transitory computer-readable medium storing instructions thereon that, when executed by one or more processors, cause the one or more processors to:
receive scan data associated with a three-dimensional scan of a patient, the three-dimensional scan representing at least a portion of a planning target volume, PTV;
determine a treatment plan involving delivery of energy from a plurality of gantry positions relative to the patient, where each gantry position corresponds to a field according to which at least a portion of the energy is configured to be delivered to the patient;
determine, for each field, a spot path configuration configured to target the planning target volume such that each spot path of a beam configuration is separated from at least one other spot path of the beam configuration by a distance that satisfies a threshold distance;
update the treatment plan based on the beam configuration for each field; and
provide control data associated with the treatment plan to a proton beam system, the control data configured to control the proton beam system when executing the treatment plan.

14. The non-transitory computer-readable medium of claim 13, wherein the instructions that cause the one or more processors to determine the beam configuration for each field cause the one or more processors to:
assign each spot path of the beam configuration to a position within the field such that energy delivered to the patient by a first spot path of the beam configuration does not overlap with energy delivered to the patient by a second spot path of the beam configuration;
and/or:
wherein the instructions that cause the one or more processors to determine the beam configuration for each field cause the one or more processors to:
assign each spot path of the beam configuration to a position within the field such that energy delivered to the patient by a first spot path of the beam configuration does not overlap with the energy delivered to the patient by a second spot path outside of a threshold distance associated with the PTV.

15. The non-transitory computer-readable medium of claim 13 or claim 14, wherein the instructions that cause the one or more processors to determine the beam configuration for each field cause the one or more processors to:
assign a plurality of spot paths to the beam configuration such that energy delivered to the patient by each spot path targets the PTV;
determine a set of spot paths that are configured to deliver energy to a location outside of a threshold distance associated with the PTV; and
unassign a subset of spot paths included in the set of spot paths from the plurality of spot paths such that energy delivered by the plurality of spot paths does not overlap at the location outside of the threshold distance.
